# EUROPÄISCHE PATENTSCHRIFT

(11) **EP 2 635 544 B1**
(45) Veröffentlichungstag und Bekanntmachung des Hinweises auf die Patenterteilung: **17.12.2014**
(21) Anmeldenummer: 11776160.1
(22) Anmeldetag: 27.10.2011
(51) Int. Cl.: C07C 5/11, B01J 23/885

(54) **VERFAHREN ZUR HERSTELLUNG EINES PHENYLCYCLOHEXANS**
METHOD FOR THE PRODUCTION OF A PHENYLCYCLOHEXANE
PROCÉDÉ DE PRODUCTION D'UN PHÉNYLCYCLOHEXANE

(30) Priorität: 02.11.2010 EP 10189628
(43) Veröffentlichungstag der Anmeldung: 11.09.2013
(73) Patentinhaber: BASF SE, 67056 Ludwigshafen (DE)
(72) Erfinder: GRALLA, Gabriele, 68161 Mannheim (DE); HEYDRICH, Gunnar, 67117 Limburgerhof (DE)
(86) Internationale Anmeldenummer: PCT/EP2011/068810
(87) Internationale Veröffentlichungsnummer: WO 2012/059387

(56) Entgegenhaltungen:
- EP-A1- 0 394 842
- WO-A1-2009/013192
- DE-C- 937 950
- US-A- 3 387 048
- US-A- 5 334 790

## Beschreibung

Die vorliegende Erfindung betrifft ein verbessertes Verfahren zur Herstellung eines substituierten oder unsubstituierten Phenylcyclohexans durch katalytische Hydrierung eines substituierten oder unsubstituierten Biphenyls.

Die Herstellung von Phenylcyclohexan durch vollständige Hydrierung eines Phenylringes des Biphenyls ist prinzipiell bekannt.

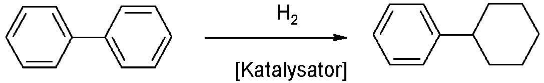

Als Nebenprodukt entsteht dabei unter anderem üblicherweise immer auch Bicyclohexyl, welches mit Phenylcyclohexan ein Azeotrop bildet.

I. Goodman beschreibt in Journal of the Chemical Society 1951, Seiten 1371-1372 die Herstellung von Phenylcyclohexan durch katalytische Hydrierung von Biphenyl zu Phenylcyclohexan in Ethanol in Gegenwart von Raney Nickel.

In Tetrahedron Letters 2000, No. 41, Seiten 5865-5868 ist die Herstellung von Phenylcyclohexan durch Hydrierung von Biphenyl in Gegenwart von Raney-Nickel-Aluminium Legierungen bei einer Temperatur von 90°C beschrieben.

DE 937 950 offenbart ein Verfahren zur katalytischen Hydrierung von Biphenyl zu Phenylcyclohexan mit einem nickel- und cobaltfreien Kupfer-Chrom Katalysator bei einer Temperatur zwischen etwa 240-260 °C.

US 3 387 048 offenbart ein Verfahren zur Herstellung von Phenylcyclohexan durch Hydrierung von Biphenyl unter Zugabe des Lösungsmittels Cyclohexan. Als geeigneter Katalysator wird 5% Palladium auf Kohle beschrieben.

Die DE 2 125 473 betrifft Katalysatoren für die partielle Hydrierung von Biphenylderivaten, die Kobaltoxid oder ein Gemisch von Kobaltoxiden wie z.B. Co₃O₄ und CoO enthalten.

Die WO 93/16972 betrifft Katalysatoren für die Hydrierung von polycyclischen und monocyclischen Aromaten wie alkylsubstituierte Benzolderivate und substitiuerten Biphenylen, wobei die Katalysatoren Ruthenium enthalten.

CN 1800121 offenbart ein Verfahren zur Herstellung von Phenylcyclohexan durch Hydrierung von Biphenyl in Gegenwart von Nickel-Aluminium Katalysatoren.

EP 0 394 842 betrifft Katalysatoren für die Hydrierung aliphatischer ungesättigter Verbindungen, wobei die Katalysatoren Nickel und Kupfer enthalten und gekennzeichnet sind durch einen Gehalt von 20 bis 75 Gew.-% Nickeloxid, 10 bis 75 Gew.-% Zirkoniumdioxid und 5 bis 50 Gew.-% Kupferoxid, jeweils bezogen auf den oxidischen, nicht reduzierten Katalysator. Die Katalysatoren eignen sich insbesondere für die Hydrierung der technisch wichtigen Verbindungen Butin-2-diol-1,4, Buten-2-diol-1,4 und 2-Ethylhexen-2-al.

Die vorangehend beschriebenen Verfahren zur Herstellung von substituiertem oder unsubstituiertem Phenylcyclohexan verwenden teilweise Katalysatoren, die entweder großtechnisch nicht einfach handhabbar oder nicht leicht verfügbar sind. Weiterhin weisen die bekannten Verfahren in einigen Fällen eine nicht zufriedenstellende Selektivität bezüglich des Zielproduktes und/oder eine zu geringe Raum-Zeit-Ausbeute auf.

Ausgehend von diesem Stand der Technik bestand die Aufgabe der vorliegenden Erfindung darin, ein Verfahren bereitzustellen, das die Herstellung von Phenylcyclohexan durch Hydrierung von Biphenyl unter wirtschaftlich optimierten Bedingungen ermöglicht. Das Verfahren soll im technischen Maßstab auf verfahrenstechnisch gut handhabbare Weise durchführbar sein und in hoher chemischer Ausbeute unter Einsatz wohlfeiler Katalysatoren zum gewünschten Produkt in hoher Selektivität führen. Die Bildung von Bicyclohexyl soll so weit wie möglich vermieden werden, da wie eingangs bereits erwähnt Bicyclohexyl und Phenylcyclohexan ein Azeotrop bilden, was die Ausbeute an reinem Phenylcyclohexan bei einer destillativen Aufreinigung erniedrigt.

Diese Aufgabe wird durch ein Verfahren zur Herstellung eines substituierten oder unsubstituierten Phenylcyclohexans der Formel I 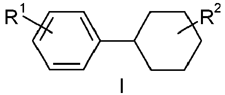 durch katalytische Hydrierung eines substituierten oder unsubstituierten Biphenyls der Formel II 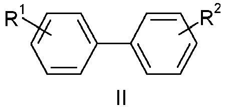 worin R¹ gleich Wasserstoff und R² gleich Wasserstoff oder Phenyl ist, oder R¹ für einen C₁-C₄-Alkylrest steht und R² gleich Wasserstoff ist, oder R¹ und R² identisch sind und jeweils für Phenyl oder den gleichen C₁-C₄-Alkylrest stehen, wobei R¹ und R² sich beide zugleich an den jeweiligen ortho-, meta- oder para-Positionen der beiden Phenylringe des Biphenyls der Formel II befinden,

in Gegenwart von Wasserstoff und einem Katalysator, umfassend
- 30 bis 70 Gew.-% sauerstoffhaltige Verbindungen des Nickels, berechnet als NiO,
- 15 bis 45 Gew.-% sauerstoffhaltige Verbindungen des Zirkons, berechnet als ZrO₂,
- 5 bis 30 Gew.-% sauerstoffhaltige Verbindungen des Kupfers, berechnet als CuO,
- 0,1 bis 10 Gew.-% sauerstoffhaltige Verbindungen des Molybdäns, berechnet als MoO₃ und
- 0 bis 10 Gew.-% weiterer Komponenten,
wobei sich die Angaben in Gew.-% auf den trockenen, nicht reduzierten Katalysator beziehen, gelöst.

In dem erfindungsgemäßen Verfahren wird als Ausgangsstoff ein Biphenyl der Formel II eingesetzt, 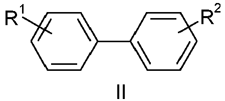 worin R¹ gleich Wasserstoff und R² gleich Wasserstoff oder Phenyl ist, oder R¹ für einen C₁-C₄-Alkylrest steht und R² gleich Wasserstoff ist, oder R¹ und R² identisch sind und jeweils für Phenyl oder den gleichen C₁-C₄-Alkylrest stehen, wobei R¹ und R² sich beide zugleich an den jeweiligen ortho-, meta- oder para-Positionen der beiden Phenylringe des Biphenyls der Formel II befinden.

Bei dem C₁-C₄-Alkylrest handelt es sich um Methyl, Ethyl, n-Propyl, Isopropyl, n-Butyl, sec.-Butyl, tert.-Butyl und 2-Methyl-1-propyl, bevorzugt Methyl und tert.-Butyl, insbesondere Methyl.

Beispiele für Biphenyle der Formel II sind unter anderem

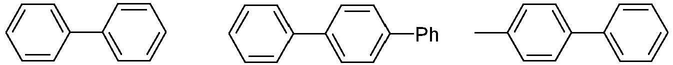

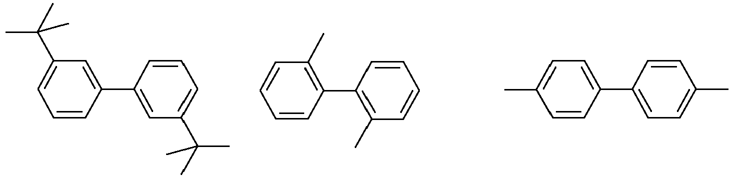

Bevorzugt handelt es sich bei der dem Biphenyl der Formel II um unsubstituiertes Biphenyl, das heißt R¹ und R² sind jeweils gleich Wasserstoff.

Bevorzugt ist daher ein erfindungsgemäßer Verfahren wie vorangehend beschrieben, worin R¹ und R² gleich Wasserstoff sind, das heißt ein Verfahren zur Herstellung von Phenylcyclohexan der Formel la 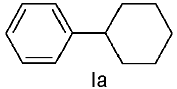 durch katalytische Hydrierung von unsubstituiertem Biphenyl der Formel IIa.

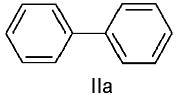

Die im erfindungsgemäßen Verfahren einsetzbaren Biphenyle der Formel II sind üblicherweise kommerziell erhältlich wie beispielsweise einfaches Biphenyl, welches aus den destillierten Ölen des Steinkohleteers gewonnen wird. Die substituierten Biphenyle sind beispielsweise durch dem Fachmann bekannte Aryl-Aryl-Kupplungsreaktionen, wie beispielsweise der Suziki-Kupplung, zugänglich.

Das erfindungsgemäße Verfahren wird in Gegenwart von Wasserstoff und in Gegenwart eines heterogenen Katalysators durchgeführt, wobei der einzusetzende heterogene Katalysator 30 bis 70 Gew.-%, bevorzugt 40 bis 60 Gew.-% sauerstoffhaltige Verbindungen des Nickels, berechnet als NiO, 15 bis 45 Gew.-%, bevorzugt 20 bis 40 Gew.-% sauerstoffhaltige Verbindungen des Zirkons, berechnet als ZrO₂, 5 bis 30 Gew.-%, bevorzugt 10 bis 25 Gew.-% sauerstoffhaltige Verbindungen des Kupfers, berechnet als CuO und 0,1 bis 10 Gew.-%, bevorzugt 0,5 bis 5 Gew.-% sauerstoffhaltige Verbindungen des Molybdäns, berechnet als MoO₃ gegebenenfalls neben weiteren Komponenten in einer Menge von 0 bis 10 Gew.-%, bevorzugt 0 bis 5 Gew.-% wie beispielsweise Graphit enthält. Dabei beziehen sich die Angaben in Gew.-% auf den trockenen, nicht reduzierten Katalysator.

Da sich die Konzentrationsangaben jeweils - falls nicht anders angegeben - auf die katalytisch aktive Masse des Katalysators beziehen, wird die katalytisch aktive Masse des Katalysators im folgenden als die Summe der Massen der katalytisch aktiven Bestandteile Zirkonium, Nickel, Kupfer und Molybdän im Katalysator, jeweils berechnet als ZrO₂, NiO, CuO bzw. MoO₃, nach dessen letzter Wärmebehandlung und vor dessen Reduktion mit Wasserstoff, definiert.

Im Rahmen einer bevorzugten Ausführungsform setzt man zur Durchführung des erfindungsgemäßen Verfahrens solche Katalysatoren ein, umfassend
- 45 bis 55 Gew.-% sauerstoffhaltige Verbindungen des Nickels, berechnet als NiO,
- 25 bis 35 Gew.-% sauerstoffhaltige Verbindungen des Zirkons, berechnet als ZrO₂,
- 13 bis 20 Gew.-% sauerstoffhaltige Verbindungen des Kupfers, berechnet als CuO,
- 1 bis 3 Gew.-% sauerstoffhaltige Verbindungen des Molybdäns, berechnet als MoO₃ und
- 0 bis 5 Gew.-% weiterer Komponenten,
wobei sich die Angaben in Gew.-% zu 100 Gew.-% ergänzen und sich auf den trockenen, nicht reduzierten Katalysator beziehen. Erfindungsgemäß insbesondere bevorzugt sind solche Katalysatoren, die aus den vorstehend genannten Komponenten in den ebenfalls vorstehend genannten Gewichtsanteilen bestehen.

Ein zum Einsatz im Rahmen des erfindungsgemäßen Verfahrens insbesondere bevorzugter Katalysator besteht zu 49 bis 53 Gew.-% aus NiO, zu 15 bis 19 Gew.-% aus CuO, zu 28 bis 32 Gew.-% aus ZrO₂ und zu 1 bis 2 Gew.-% aus MoO₃ sowie gegebenenfalls zu 0 bis 3 Gew.-% aus weiteren Komponenten wie beispielsweise Graphit, wobei sich die jeweils gewählten Gewichtsanteile der einzelnen Komponenten auf den trockenen, nicht reduzierten Katalysator beziehen und zu 100 Gew.-% ergänzen. Derartige Katalysatoren sind bekannt und können beispielsweise wie in der EP 0 696 572 beschrieben hergestellt werden.

Die erfindungsgemäß einsetzbaren Katalysatoren können z.B. durch Einsatz von Fällungsmethoden hergestellt werden. So können sie beispielsweise durch eine gemeinsame Fällung der Nickel- und Kupferkomponenten aus einer diese Elemente enthaltenden, wässrigen Salzlösung mittels Mineralbasen in Gegenwart einer Aufschlämmung einer schwerlöslichen, sauerstoffhaltigen Zirkoniumverbindung und anschließendes Waschen, Trocknen und Kalzinieren des erhaltenen Präzipitats erhalten werden. Als schwerlösliche, sauerstoffhaltige Zirkoniumverbindungen können beispielsweise Zirkoniumdioxid, Zirkoniumoxidhydrat, Zirkoniumphosphate, -borate und -silikate Verwendung finden. Die Aufschlämmungen der schwerlöslichen Zirkoniumverbindungen können durch Suspendieren feinkörniger Pulver dieser Verbindungen in Wasser unter kräftigem Rühren hergestellt werden. Vorteilhaft werden diese Aufschlämmungen durch Ausfällen der schwerlöslichen Zirkoniumverbindungen aus wässrigen Zirkoniumsalzlösungen mittels Mineralbasen erhalten.

Bevorzugt werden die erfindungsgemäß einsetzbaren Katalysatoren über eine gemeinsame Fällung (Mischfällung) aller ihrer Komponenten hergestellt. Dazu wird zweckmäßigerweise eine die Katalysatorkomponenten enthaltende, wässrige Salzlösung in der Wärme und unter Rühren so lange mit einer wässrigen Mineralbase, insbesondere einer Alkalimetallbase - beispielsweise Natriumcarbonat, Natriumhydroxid, Kaliumcarbonat oder Kaliumhydroxid - versetzt, bis die Fällung vollständig ist. Die Art der verwendeten Salze ist im Allgemeinen nicht kritisch - da es bei dieser Vorgehensweise vornehmlich auf die Wasserlöslichkeit der Salze ankommt, ist ein Kriterium ihre zur Herstellung dieser verhältnismäßig stark konzentrierten Salzlösungen erforderliche, gute Wasserlöslichkeit. Es wird als selbstverständlich erachtet, dass bei der Auswahl der Salze der einzelnen Komponenten natürlich nur Salze mit solchen Anionen gewählt werden, die nicht zu Störungen führen, sei es, indem sie unerwünschte Fällungen verursachen oder indem sie durch Komplexbildung die Fällung erschweren oder verhindern.

Erfindungsgemäß einsetzbare Katalysatoren mit besonders vorteilhaften Eigenschaften sind dadurch erhältlich, dass man einen Teil der Zirkoniumkomponente des Katalysators, zweckmäßigerweise aus einer wässrigen Zirkoniumsalzlösung, separat in einer Fällungsapparatur durch Zugabe wässriger Mineralbasen fällt. Auf das so erhaltene, vorzugsweise frisch gefällte Zirkoniumoxid-Hydrat, kann dann der restliche Teil der Zirkoniumkomponente des Katalysators zusammen mit den anderen katalytisch aktiven Komponenten in einer Mischfällung gefällt werden, wie oben beschrieben wurde. Dabei erweist es sich in der Regel als besonders zweckmäßig 10 bis 80 Gew.%, vorzugsweise 30 bis 70 Gew.-% und insbesondere 40 bis 60 Gew.-% der Gesamtzirkoniummenge der katalytisch aktiven Masse vorzufällen.

Die bei diesen Fällungsreaktionen erhaltenen Niederschläge sind im Allgemeinen chemisch uneinheitlich und bestehen u.a. aus Mischungen der Oxide, Oxidhydrate, Hydroxide, Carbonate und unlöslichen und basischen Salze der genannten Metalle. Es kann sich für die Filtrierbarkeit der Niederschläge als günstig erweisen, wenn sie gealtert werden, d.h. wenn man sie noch einige Zeit nach der Fällung, gegebenenfalls in Wärme oder unter Durchleiten von Luft, sich selbst überlässt.

Die nach diesen Fällungsverfahren erhaltenen Niederschläge können wie üblich zu den erfindungsgemäß einsetzbaren Katalysatoren weiterverarbeitet werden. Nach dem Waschen werden sie im Allgemeinen bei 80 bis 200°C, vorzugsweise bei 100 bis 150°C, getrocknet und danach kalziniert. Die Kalzinierung wird im Allgemeinen bei Temperaturen zwischen 300 und 800°C, vorzugsweise bei 400 bis 600°C, insbesondere bei 450 bis 550°C ausgeführt.

Nach der Kalzinierung wird der Katalysator zweckmäßigerweise konditioniert, sei es, dass man ihn durch Vermahlen auf eine bestimmte Korngröße einstellt oder dass man ihn nach seiner Vermahlung mit Formhilfsmitteln wie Graphit oder Stearinsäure vermischt, mittels einer Tablettenpresse zu Formlingen verpresst und tempert. Die Temperaturen entsprechen dabei im Allgemeinen den Temperaturen bei der Kalzinierung.

Die auf diese Weise hergestellten Katalysatoren enthalten die katalytisch aktiven Metalle in Form eines Gemisches ihrer sauerstoffhaltigen Verbindungen, d.h. insbesondere als Oxide und Mischoxide.

Die auf diese Weise hergestellten Katalysatoren können als solche gelagert und eingesetzt werden. Vor ihrem Einsatz als Katalysatoren im Rahmen des erfindungsgemäßen Verfahrens werden sie üblicherweise vorreduziert. Sie können jedoch auch ohne Vorreduktion eingesetzt werden, wobei sie dann unter den Bedingungen der erfindungsgemäßen Hydrierung durch den im Reaktor vorhandenen Wasserstoff reduziert werden. Zur Vorreduktion werden die Katalysatoren im Allgemeinen zunächst bei 150 bis 200°C über einen Zeitraum von 12 bis 20 Stunden einer Stickstoff-WasserstoffAtmosphäre ausgesetzt und anschließend noch bis zu ca. 24 Stunden bei 200 bis 300°C in einer Wasserstoffatmosphäre behandelt. Bei dieser Vorreduktion wird ein Teil der in den Katalysatoren vorliegenden sauerstoffhaltigen Metallverbindungen üblicherweise zu den entsprechenden Metallen reduziert, so dass diese gemeinsam mit den verschiedenartigen Sauerstoffverbindungen in der aktiven Form des Katalysators vorliegen.

Im Allgemeinen werden die erfindungsgemäß verwendeten Katalysatoren bevorzugt in Form von Vollkatalysatoren eingesetzt. Mit dem Begriff "Vollkatalysator" wird ein Katalysator bezeichnet, der im Gegensatz zu einem Trägerkatalysator nur aus katalytisch aktiver Masse besteht. Vollkatalysatoren können dergestalt eingesetzt werden, dass man die katalytisch aktive, zu Pulver vermahlene Masse in das Reaktionsgefäß einbringt oder, dass man die katalytisch aktive Masse nach Mahlung, Vermischung mit Formhilfsmitteln, Formung und Temperung als Katalysatorformkörper - beispielsweise als Kugeln, Zylinder, Tabletten, Ringe, Spiralen, Stränge und dergleichen mehr - im Reaktor anordnet.

Im Rahmen einer bevorzugten Ausführungsform des erfindungsgemäßen Verfahrens setzt man in der katalytischen Hydrierung, das heißt in dem Hydrierschritt des erfindungsgemäßen Verfahrens, den gewählten Katalysator, der ein heterogener Katalysator ist, in Form eines Festbettkatalysators ein.

Zur Durchführung des erfindungsgemäßen Verfahrens bringt man den wie vorstehend beschriebenen Ausgangsstoff, nämlich ein Biphenyl der Formel II, mit Wasserstoff und dem gewählten Katalysator in Kontakt. Der Wasserstoff kann dabei in unverdünnter Form, üblicherweise in einer Reinheit von etwa 99,9 Vol.-% oder in verdünnter Form, d.h. in Form von Gemischen mit inerten Gasen wie beispielsweise Stickstoff oder Argon, eingesetzt werden. Bevorzugt setzt man Wasserstoff in unverdünnter Form ein.

Die katalytische Hydrierung kann mit gutem Erfolg ohne Zusatz von Lösungsmittel oder in Gegenwart von unter den Reaktionsbedingungen inerten organischen Lösungsmitteln wie beispielsweise Methanol, Ethanol, Isopropanol, Hexan, Heptan, Cyclohexan und dergleichen mehr durchgeführt werden. Bevorzugt führt man die Hydrierung ohne Zusatz von Lösungsmittel durch.

In dem erfindungsgemäßen Verfahren kann die Hydrierung des Biphenyls der Formel II bei einem Wasserstoffdruck (absolut) im Bereich von 1 bis 200 bar, bevorzugt von 2 bis 150 bar, besonders bevorzugt von 4 bis 80 bar, ganz besonders bevorzugt von 5 bis 50 bar durchgeführt werden. Als Reaktionstemperatur zur Durchführung der Hydrierung wählt man vorteilhaft eine Temperatur im Bereich von 50 bis 250°C, bevorzugt von 100 bis 180°C, ganz besonders bevorzugt von 110 bis 160°C.

Praktisch geht man bei der Durchführung im Allgemeinen so vor, dass man dem Katalysator, der sich üblicherweise in einem bevorzugt von außen beheizten Festbettreaktor wie beispielsweise einem Rohrreaktor, Autoklaven oder Rohrbündelreaktor befindet, bei der gewünschten Reaktionstemperatur und dem gewünschten Druck das umzusetzende Biphenyl der Formel II zuführt. Dabei belastet man den Katalysator im Allgemeinen mit 0,1 bis 1,0 kg, bevorzugt mit 0,1 bis 0,6 kg und besonders bevorzugt mit 0,2 bis 0,4 kg des Biphenyls der Formel II pro kg Katalysator und pro Stunde. Hierbei kann es zweckmäßig sein, das einzusetzende Biphenyl der Formel II bereits vor der Zuführung in das Reaktionsgefäß bzw. den Reaktor zu erwärmen und zwar bevorzugt auf die Reaktionstemperatur.

Der Reaktor kann sowohl in der Sumpf- als auch in der Rieselfahrweise betrieben werden, d.h. man kann die Ausgangsstoffe sowohl von unten nach oben als auch von oben nach unten durch den Reaktor leiten. Der Hydrierschritt in dem erfindungsgemäßen Verfahren kann sowohl diskontinuierlich als auch kontinuierlich durchgeführt werden. In beiden Fällen kann nicht umgesetztes Edukt zusammen mit dem Wasserstoff im Kreis geführt werden.

Der Hydrierschritt in dem erfindungsgemäßen Verfahren kann auch stufenweise in einer Kaskade von mehreren, d.h. 2 bis in der Regel 4, bevorzugt 2 oder 3 und insbesondere bevorzugt in zwei hintereinander geschalteten Reaktoren, bevorzugt Festbettreaktoren durchgeführt werden. Dabei wird in dem ersten, üblicherweise als Hauptreaktor bezeichneten Reaktor unter den vorstehend beschriebenen Reaktionsbedingungen der Hauptumsatz der Reaktion erzielt und das erhaltene Rohprodukt einem zweiten, üblicherweise als Nachreaktor bezeichneten Reaktor zugeführt, in dem das noch nicht umgesetzte Ausgangsmaterial in erfindungsgemäßer Weise zumindest weitgehend zum Phenylcyclohexan der Formel I überführt wird. Dabei können die Reaktionsbedingungen unabhängig voneinander vorzugsweise in den vorstehend genannten Bereichen gewählt werden.

In dem erfindungsgemäßen Verfahren kann die Hydrierung diskontinuierlich, halb- oder vollkontinuierlich durchgeführt werden. Bevorzugt führt man die katalytische Hydrierung in dem erfindungsgemäßen Verfahren kontinuierlich, insbesondere vollkontinuierlich durch, wobei die Ausgangsstoffe kontinuierlich in den Reaktor eingetragen und das erhaltenen Reaktionsgemisch bzw. Reaktionsprodukt kontinuierlich aus dem Reaktor ausgetragen werden.

Das im Hydrierschritt des erfindungsgemäßen Verfahrens erhaltene Reaktionsgemisch bzw. Reaktionsprodukt kann nach Zwischenpufferung in einem Behälter durch fraktionierte Destillation, bevorzugt durch fraktionierte Destillation im Vakuum aufgereinigt werden, um ein substituiertes oder unsubstituiertes Phenylcyclohexan der Formel I in einer Reinheit von größer 97 Gew.-%, bevorzugt größer 98 Gew.-% bezogen auf die Gesamtmasse zu erhalten. Die destillative Aufarbeitung des erhaltenen Reaktionsproduktes kann in einer batchweise oder kontinuierlich betriebenen Rektifikationskolonne durchgeführt werden. Verschiedene technische Ausführungen solcher Destillationskolonnen, die eine diskontinuierliche, halbkontinuierlice oder vollkontinuierliche Destillation erlauben, sind in der einschlägigen Literatur beschrieben.

Eine bevorzugte Variante des erfindungsgemäßen Verfahrens zeichnet sich demnach dadurch aus, dass in einem weiteren Verfahrensschritt ein bei der katalytischen Hydrierung erhaltenes Reaktionsprodukt zur weiteren Aufreinigung des substituierten oder unsubstituierten Phenylcyclohexans der Formel I destillativ aufgearbeitet wird.

Für die kontinuierliche destillative Zerlegung von Mehrstoffgemischen sind nach dem Stand der Technik verschiedene Verfahrensvarianten gebräuchlich. Im einfachsten Fall wird das Zulaufgemisch in zwei Fraktionen, eine leichtsiedende Kopffraktion und eine schwersiedende Sumpffraktion, zerlegt. Bei der Auftrennung von Zulaufgemischen in mehr als zwei Fraktionen müssen nach dieser Verfahrensvariante mehrere Destillationskolonnen eingesetzt werden. Um den apparativen Aufwand zu begrenzen, setzt man bei der Auftrennung von Vielstoffgemischen nach Möglichkeit Kolonnen mit flüssigen oder dampfförmigen Seitenabzügen ein. Die Anwendungsmöglichkeit von Destillationskolonnen mit Seitenabzügen ist jedoch dadurch stark eingeschränkt, dass nach dem Stand der Technik die an den Seitenabzugsstellen entnommenen Produkte nie völlig rein sind. Bei Seitenentnahmen im Verstärkungsteil, die üblicherweise in flüssiger Form erfolgen, enthält das Seitenprodukt noch Anteile an leichtsiedenden Komponenten, die über Kopf abgetrennt werden sollen. Entsprechendes gilt für Seitenentnahmen im Abtriebsteil, die meist dampfförmig erfolgen, bei denen das Seitenprodukt noch Hochsiederanteile aufweist. Die Verwendung von konventionellen Seitenabzugskolonnen ist daher auf Fälle begrenzt, in denen verunreinigte Seitenprodukte zulässig sind.

Eine Abhilfemöglichkeit bieten Trennwandkolonnen. Dieser Kolonnentyp ist beispielsweise beschrieben in US 2,471,134; US 4,230,533; EP 0 122 367; EP 0 126 288; EP 0 133 510; Chem. Eng. Technol. 10 (1987) 92 - 98; Chem.-Ing.-Tech. 61 (1989) Nr.1, 16 - 25; Gas Separation and Purification 4 (1990) 109 - 114; Process Engineering 2 (1993) 33 - 34; Trans IChemE 72 (1994) Part A 639 - 644 und Chemical Engineering 7 (1997) 72 - 76.

Die destillative Aufarbeitung des Reaktionsproduktes aus der katalytischen Hydrierung erfolgt vorteilhaft im Vakuum bei Absolutdrücken zwischen 1 und 100 mbar, bevorzugt 5 bis 50 mbar. Bei den Destillationen im Vakuum werden bevorzugt Destillationskolonnen mit geordneten Gewebepackungen mit einer spezifischen Oberfläche zwischen 250 und 1000 m²/m³ eingesetzt.

Bei einer fraktionierten Batchdestillation eines aus Biphenyl gewonnenen Reaktionsaustrages, der bereits das unerwünschte Überhydrierprodukt Bicyclohexyl enthält, kann in einer oder mehrerer leichtsiedenden Fraktionen Bicyclohexyl abgetrennt werden, das von nennenswerten Anteilen des gewünschten Phenylcyclohexans begleitet ist, mit dem es ein Leichtsiedeazeotrop ausbildet. Ist das Bicyclohexyl aus der Destillationsblase abdestilliert, können mittelsiedende Reinfraktionen an Phenylcyclohexan gewonnen werden. Im Kolonnensumpf verbleibt das nicht umgesetzte Edukt Biphenyl, das je nach vorhandener Menge und technischer Gegebenheit auch noch zum Teil abdestilliert werden kann.

Diesen Effekt kann man sich in der Praxis zunutze machen, indem die Hydrierreaktion nicht zum vollständigen Umsatz des Biphenyls durchgeführt wird, sondern vorher unterbrochen wird, sobald erste Mengen an Bicyclohexyl im Reaktionsaustrag gefunden werden. Dies kann beispielsweise bei einer Batchreaktion durch eine Begrenzung der Reaktionszeit oder bei einer kontinuierlich durchgeführten Reaktion durch Regelung der Reaktortemperatur oder des Hydrierdruckes erzielt werden, das heißt, dass der Umsatz an Edukt durch Regelung von Reaktionstemperatur und/oder Reaktionsdruck und/oder Verweilzeit der Reaktionslösung im Hydrierapparat gesteuert werden kann.

In einer bevorzugten Variante des erfindungsgemäßen Verfahrens wird die Hydrierung so durchgeführt, dass zwischen 90 und 95 % des substituierten oder unsubstituierten Biphenyls der Formel II umgesetzt werden.

Der nach der fraktionierten Destillation verbliebene, mit Biphenyl angereicherte Sumpf kann wiederum in die Reaktion zurückgeführt werden, wodurch die Gesamtausbeute an Phenylcyclohexan erhöht werden kann.

In einer besonders bevorzugten Form des erfindungsgemäßen Verfahrens werden Fraktionen, die bei der destillativen Aufarbeitung des Reaktionsproduktes aus der katalytischen Hydrierung anfallen und die noch Biphenyl der Formel II enthalten, ganz oder teilweise in die Hydrierung zurückgeführt.

Die Erfindung wird durch folgende, die Erfindung jedoch nicht einschränkende Beispiele erläutert.

### Beispiele

Alle Experimente wurden in einem 300 ml HC-Laborautoklav durchgeführt, der mit einem Katalysatorkorb versehen war. Über ein seitlich angesetztes Rohr war eine Probenentnahme möglich. Die Temperaturregelung erfolgte über ein externes Ölbad.

### Analytik

GC-Analytik wurde nach der folgenden Methode durchgeführt: 30 m DB-WAX, ID.: 0,2 mm, FD. 0,5 µm, Initial Temp.: 200 °C, Det. Temp.: 250 °C; Start 80 °C - 3 °C / min - 200 °C / 15 min; auf 240 °C / 20 min isotherm; Einspritzmenge: 0,2 µl; Trägergas He; t_{R} = min; t_{R} (Biphenyl): 25,6; t_{R} (Phenylcyclohexan): 15,4; t_{R} (Phenylcyclohexen): 10,2, 10,6 und 11,0; t_{R} (Bicyclohexyl): 8,7.

### Beispiel 1

In einem 300 ml Laborautoklaven wurden 99,6 g Biphenyl in Gegenwart von 5 g eines Katalysators bestehend aus 50 Gew.-% NiO, 17 Gew.-% CuO, 30,5 Gew.-% ZrO₂ und 1,5 Gew.-% MoO₃ in Form von Tabletten mit einem Durchmesser und einer Höhe von jeweils 3 mm bei einem Wasserstoffdruck von 30 bar und einer Temperatur von 130 °C unter Rühren hydriert. Nach 22 h Reaktionsdauer wurde der Katalysator abfiltriert. Das erhaltene Reaktionsgemisch wurde zu den in Tabelle 1 angegebenen Zeiten gaschromatographisch analysiert. Man erhielt die in Tabelle 1 angegebenen Ergebnisse.

**Tabelle 1**

| Laufzeit [h] | Biphenyl | Phenylcyclohexan | Phenylcyclohexen | Bicyclohexyl | Temperatur [°C] |
|---|---|---|---|---|---|
| | GC-FI.% | | | | |
| 2 | 98,78 | 1,21 | 0 | 0 | 90 |
| 3 bis 6 | 94,88 | 5,12 | 0 | 0 | 90 |
| 7 bis 12 | 64,78 | 35,08 | 0 | 0,14 | 120 |
| 13 bis 18 | 22,76 | 76,47 | 0 | 0,77 | 130 |
| 19 bis 21 | 1,05 | 95,93 | 0,19 | 2,83 | 130 |
| 22 | 0 | 93,76 | 0,31 | 5,93 | 130 |

### Beispiel 2

In einem 300 ml Laborautoklaven wurden 100 g Biphenyl in Gegenwart von 5 g eines Katalysators bestehend aus 50 Gew.-% NiO, 17 Gew.-% CuO, 30,5 Gew.-% ZrO₂ und 1,5 Gew.-% MoO₃ in Form von Tabletten mit einem Durchmesser und einer Höhe von jeweils 3 mm bei einem Wasserstoffdruck von 40 bar und einer Temperatur von 130 °C unter Rühren hydriert. Nach 14 h Reaktionsdauer wurde der Katalysator abfiltriert. Das erhaltene Reaktionsgemisch wurde zu den in Tabelle 2 angegebenen Zeiten gaschromatographisch analysiert. Man erhielt die in Tabelle 2 angegebenen Ergebnisse (jeweils in GC-Flächen-%).

**Tabelle 2**

| Laufzeit [h] | Biphenyl | Phenylcyclohexan | Phenylcyclohexen | Bicyclohexyl | Temperatur [°C] |
|---|---|---|---|---|---|
| | GC-FI.% | | | | |
| 5 | 65,17 | 34,36 | 0 | 0,46 | 130 |
| 11 | 21,14 | 77,52 | 0,11 | 1,22 | 130 |
| 13 | 4,31 | 93,10 | 0,16 | 2,46 | 130 |
| 14 | 0,92 | 94,93 | 0,20 | 3,94 | 130 |
| Austrag | 0 | 94,83 | 0,18 | 4,98 | - |

### Beispiel 3

In einem 300 ml Laborautoklaven wurden 100 g Biphenyl in Gegenwart von 5 g eines Katalysators bestehend aus 50 Gew.-% NiO, 17 Gew.-% CuO, 30,5 Gew.-% ZrO₂ und 1,5 Gew.-% MoO₃ in Form von Tabletten mit einem Durchmesser und einer Höhe von jeweils 3 mm bei einem Wasserstoffdruck von 10 bar und einer Temperatur von 150 °C unter Rühren hydriert. Nach 17 h Reaktionsdauer wurde der Katalysator abfiltriert. Das erhaltene Reaktionsgemisch wurde zu den in Tabelle 3 angegebenen Zeiten gaschromatographisch analysiert. Man erhielt die in Tabelle 3 angegebenen Ergebnisse (jeweils in GC-Flächen-%).

**Tabelle 3**

| Laufzeit [h] | Biphenyl | Phenylcyclohexan | Phenylcyclohexen | Bicyclohexyl | Temperatur [°C] |
|---|---|---|---|---|---|
| | GC-FI.% | | | | |
| 6 | 71,73 | 27,87 | 0 | 0,31 | 150 |
| 11 | 41,39 | 58,10 | 0 | 0,43 | 150 |
| 17 | 4,56 | 94,13 | 0 | 0,12 | 150 |
| Austrag | 0,12 | 95,43 | 0,24 | 4,04 | - |

### Beispiel 4

In einem 300 ml Laborautoklaven wurden 100 g Biphenyl in Gegenwart von 5 g eines Katalysators bestehend aus 50 Gew.-% NiO, 17 Gew.-% CuO, 30,5 Gew.-% ZrO₂ und 1,5 Gew.-% MoO₃ in Form von Tabletten mit einem Durchmesser und einer Höhe von jeweils 3 mm bei einem Wasserstoffdruck von 10 bar und einer Temperatur von 130 °C unter Rühren hydriert. Nach 48 h Reaktionsdauer wurde der Katalysator abfiltriert. Das erhaltene Reaktionsgemisch wurde zu den in Tabelle 4 angegebenen Zeiten gaschromatographisch analysiert. Man erhielt die in Tabelle 4 angegebenen Ergebnisse (jeweils in GC-Flächen-%).

**Tabelle 4**

| Laufzeit [h] | Biphenyl | Phenylcyclohexan | Phenylcyclohexen | Bicyclohexyl | Temperatur [°C] |
|---|---|---|---|---|---|
| | GC-FI.% | | | | |
| 9 | 85,62 | 14,29 | 0 | 0 | 130 |
| 15 | 72,38 | 27,46 | 0 | 0,07 | 130 |
| 26 | 45,06 | 54,60 | 0 | 0,05 | 130 |
| 48 | 1,39 | 92,18 | 0,29 | 6,08 | 130 |
| Austrag | 0 | 92,4 | 0,22 | 7,24 | - |

### Vergleichsbeispiel 5

In einem 300 ml Laborautoklaven wurden 20 g Biphenyl, gelöst in 80 g Methanol in Gegenwart von 5 g eines Katalysators bestehend aus 0,5% Ru auf Al₂O₃-Träger in Form von 4-6 mm Kugeln bei einem Wasserstoffdruck von 30 bar und einer Temperatur von 90-120 °C unter Rühren hydriert. Nach 12 h Reaktionsdauer wurde der Katalysator abfiltriert. Das erhaltene Reaktionsgemisch wurde zu den in Tabelle 5 angegebenen Zeiten gaschromatographisch analysiert. Man erhielt die in Tabelle 5 angegebenen Ergebnisse (jeweils in GC-Flächen-%).

**Tabelle 5**

| Laufzeit [h] | Biphenyl | Phenylcyclohexan | Phenylcyclohexen | Bicyclohexyl | Temperatur [°C] |
|---|---|---|---|---|---|
| | GC-FI.% | | | | |
| 1 | 96,43 | 3,57 | 0 | 0 | 90 |
| 3 | 90,49 | 8,95 | 0 | 0,56 | 90 |
| 4 bis 6 | 71,99 | 26,49 | 0 | 1,53 | 90 |
| 7 | 56,97 | 40,96 | 0 | 2,07 | 120 |
| 8 bis 11 | 12,34 | 82,00 | 0,70 | 4,96 | 120 |
| 12 | 2,49 | 87,93 | 1,14 | 8,44 | 120 |
| 13 | 0 | 86,80 | 1,53 | 11,67 | 120 |

### Vergleichsbeispiel 6

In einem 300 ml Laborautoklaven wurden 100 g Biphenyl in Gegenwart von 5 g eines Katalysators bestehend aus 0,5% Ru auf Al₂O₃-Träger in Form von 4-6 mm Kugeln bei einem Wasserstoffdruck von 30 bar und einer Temperatur von 120 °C unter Rühren hydriert. Nach 14,5 h Reaktionsdauer wurde der Katalysator abfiltriert. Das erhaltene Reaktionsgemisch wurde zu den in Tabelle 6 angegebenen Zeiten gaschromatographisch analysiert. Man erhielt die in Tabelle 6 angegebenen Ergebnisse (jeweils in GC-Flächen-%).

**Tabelle 6**

| Laufzeit [h] | Biphenyl | Phenylcyclohexan | Phenylcyclohexen | Bicyclohexyl | Temperatur [°C] |
|---|---|---|---|---|---|
| | GC-FI.% | | | | |
| 10 | 27,68 | 64,97 | 0,74 | 6,52 | 120 |
| 12 | 14,22 | 75,76 | 1,08 | 8,87 | 120 |
| 13 | 5,69 | 81,11 | 1,45 | 11,74 | 120 |
| 14,5 | 1,14 | 80,33 | 2,01 | 16,52 | 120 |

### Vergleichsbeispiel 7

In einem 300 ml Laborautoklaven wurden 100 g Biphenyl in Gegenwart von 5 g eines Katalysators bestehend aus 0,3% Ru auf SiO₂ in Form von 4-6 mm Kugeln bei einem Wasserstoffdruck von 40 bar und einer Temperatur von 130 °C unter Rühren hydriert. Nach 5 h Reaktionsdauer wurde der Katalysator abfiltriert. Das erhaltene Reaktionsgemisch wurde zu den in Tabelle 7 angegebenen Zeiten gaschromatographisch analysiert. Man erhielt die in Tabelle 7 angegebenen Ergebnisse (jeweils in GC-Flächen-%).

**Tabelle 7**

| Laufzeit [h] | Biphenyl | Phenylcyclohexan | Phenylcyclohexen | Bicyclohexyl | Temperatur [°C] |
|---|---|---|---|---|---|
| | GC-FI.% | | | | |
| 5 | 0,71 | 5,21 | 2,23 | 91,85 | 130 |
| Austrag | 0,07 | 0,09 | 0,09 | 99,56 | - |

### Vergleichsbeispiel 8

In einem 300 ml Laborautoklaven wurden 100 g Biphenyl in Gegenwart von 5 g eines Katalysators bestehend aus Ru auf SiO₂ in Form 0,3% Ru auf SiO₂ in Form von 4-6 mm Kugeln bei einem Wasserstoffdruck von 10 bar und einer Temperatur von 90 °C unter Rühren hydriert. Nach 23 h Reaktionsdauer wurde der Katalysator abfiltriert. Das erhaltene Reaktionsgemisch wurde zu den in Tabelle 8 angegebenen Zeiten gaschromatographisch analysiert. Man erhielt die in Tabelle 8 angegebenen Ergebnisse (jeweils in GC-Flächen-%).

**Tabelle 8**

| Laufzeit [h] | Biphenyl | Phenylcyclohexan | Phenylcyclohexen | Bicyclohexyl | Temperatur [°C] |
|---|---|---|---|---|---|
| | GC-FI.% | | | | |
| 5 | 78,69 | 12,34 | 0,35 | 8,29 | 90 |
| 11 | 53,27 | 31,44 | 0,63 | 14,30 | 90 |
| 17 | 29,82 | 46,39 | 0,82 | 22,70 | 90 |
| 23 | 9,92 | 53,20 | 0,98 | 35,77 | 90 |
| Austrag | 8,34 | 53,55 | 0,95 | 37,04 | - |

### Beispiel 9 Destillation von Roh-Phenylcyclohexan

In Tabelle 9 sind die Ergebnisse der Destillation des Rohaustrags mit der folgenden Zusammensetzung 80,9% Phenylcyclohexan, 18,3% Bicyclohexyl und 0,86% Biphenyl (jeweils in GC-Gew.%), der Batch-Destillation in einer 100 cm langen, schutzbeheizten Drehbandkolonne (ca. 20 theoretische Stufen) zusammengestellt. Die Apparatur war mit einem 0,1 I Doppelmantelherzkolben im Sumpf und einem Dampfteiler ausgerüstet. Das Brüdenrohr im Kopf war mit Heizband begleit beheizt, der Kondensator wurde bei 2 °C betrieben. Es wurde bei einem Rücklaufverhältnis von 5:1 fraktioniert. Im Vergleich dazu sind in der Tabelle 10 die Ergebnisse eingetragen, die bei der Destillation des Austrags mit der folgenden Zusammensetzung 94,3% Phenylcyclohexan, 5,2% Bicyclohexyl (jeweils in GC-Gew.%) erhalten wurden, der deutlich weniger Bicyclohexyl enthielt und eine deutlich größere Menge an spezifikationsgerechten Phenylcyclohexan lieferte.

Um bicyclohexylarmes Phenylcyclohexan bereitzustellen, wurden in einer Destillationskolonne DN50, die mit ca. 2,4 m Sulzer DX-Packung (ca. 48 th. Stufen) ausgerüstet war, kontinuierliche Versuche bei 10 mbar Kopfdruck durchgeführt.
Das über Sumpf gewonnene Wertprodukt enthielt jeweils über 98% Phenylcyclohexan bei Bicyclohexyl-Konzentrationen zwischen 0,2 und 0,7%. Der Bicyclohexyl-Gehalt im Destillat bewegte sich in Abhängigkeit vom Rücklaufverhältnis (4:1 bis 19:1) zwischen 19 und 50 %. Bei höheren Rücklaufverhältnissen (11:1 und mehr) konnten Destillationsausbeuten bis zu 90 % erreicht werden.

**Tabelle 9: Phenylcyclohexan Destillation diskontinuierlich.**

| Probe | Menge [g] | % Destillat | | Dicyclohexyl [RT 18,3] | | Phenylcyclohexan [RT 27,9] | | Diphenyl [RT 39,8] | Rest |
|---|---|---|---|---|---|---|---|---|---|
| | | [Fr.%] | [ges.%] | [Gew. | [FI.%] | [Gew.% | [FI.%] | [FI.%] | [FI.%] |
| Einsatz | 55,5 | | | 18,3 | 17,72 | 80,9 | 79,22 | 0,86 | 2,20 |
| Fraktion 1 | 3,7 | 7% | 7% | 44,1 | 43,73 | 53,4 | 53,39 | 0 | 2,88 |
| Fraktion 2 | 6,4 | 12% | 18% | 37,3 | 36,79 | 60,6 | 60,18 | 0 | 3,03 |
| Fraktion 3 | 5,4 | 10% | 28% | 32,5 | 32,01 | 65,5 | 64,97 | 0 | 3,02 |
| Fraktion 4 | 7,0 | 13% | 41% | 26,2 | 25,86 | 71,6 | 71,23 | 0 | 2,91 |
| Fraktion 5 | 9,7 | 17% | 58% | 16,1 | 15,72 | 82,7 | 81,89 | 0 | 2,39 |
| Fraktion 6 | 5,7 | 10% | 68% | 8,5 | 8,25 | 90,9 | 89,99 | 0,01 | 1,75 |
| Fraktion 7 | 8,1 | 15% | 83% | 2,7 | 2,49 | 98,3 | 96,60 | 0,01 | 0,90 |
| Fraktion 8 | 4,6 | 8% | 91% | 0,7 | 0,57 | 100,6 | 99,03 | 0,01 | 0,39 |
| Sumpf | 2,8 | 5% | 96% | 0,2 | 0,02 | 82,8 | 80,83 | 14,96 | 4,19 |

**Tabelle 10: Phenylcyclohexan Destillation diskontinuierlich.**

| Probe | Menge | % Destillat | | Bicyclohexyl [RT 18,3] | | Phenylcyclohexan [RT 27,9] | | Rest |
|---|---|---|---|---|---|---|---|---|
| | [g] | [Fr.%] | [ges.%] | [Gew. | [FI.%] | [Gew.% | [FI.%] | [FI.%] |
| Einsatz | 51,5 | | | 5,2 | 5,04 | 94,3 | 93,98 | 0,98 |
| Fraktion 1 | 3,6 | 7% | 7% | 24,1 | 24,00 | 75,2 | 75,58 | 0,42 |
| Fraktion 2 | 5,1 | 10% | 17% | 12,6 | 12,47 | 86,7 | 86,95 | 0,58 |
| Fraktion 3 | 5,7 | 11% | 28% | 8,2 | 8,01 | 91,4 | 91,68 | 0,31 |
| Fraktion 4 | 6,0 | 12% | 40% | 5,6 | 5,46 | 94,1 | 94,31 | 0,23 |
| Fraktion 5 | 5,6 | 11% | 50% | 3,8 | 3,60 | 95,9 | 96,20 | 0,20 |
| Fraktion 6 | 6,0 | 12% | 62% | 1,8 | 1,62 | 97,9 | 98,22 | 0,16 |
| Fraktion 7 | 9,1 | 18% | 80% | 0,7 | 0,55 | 99,0 | 99,34 | 0,11 |
| Fraktion 8 | 5,4 | 10% | 90% | 0,3 | 0,10 | 100,2 | 99,81 | 0,09 |
| Fraktion 9 | 2,8 | 5% | 96% | 0,2 | 0,05 | 99,9 | 99,87 | 0,08 |
| Sumpf | 1,9 | 4% | 99% | 0,2 | 0 | 90,1 | 90,84 | 9,16 |

## Patentansprüche

1. Verfahren zur Herstellung eines substituierten oder unsubstituierten Phenylcyclohexans der Formel I 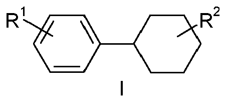 durch katalytische Hydrierung eines substituierten oder unsubstituierten Biphenyls der Formel II 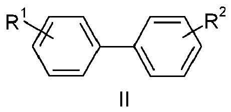 worin R¹ gleich Wasserstoff und R² gleich Wasserstoff oder Phenyl ist, oder R¹ für einen C₁-C₄-Alkylrest steht und R² gleich Wasserstoff ist, oder R¹ und R² identisch sind und jeweils für Phenyl oder den gleichen C₁-C₄-Alkylrest stehen, wobei R¹ und R² sich beide zugleich an den jeweiligen ortho-, meta- oder para-Positionen der beiden Phenylringe des Biphenyls der Formel II befinden,
in Gegenwart von Wasserstoff und einem Katalysator, umfassend
- 30 bis 70 Gew.-% sauerstoffhaltige Verbindungen des Nickels, berechnet als NiO,
- 15 bis 45 Gew.-% sauerstoffhaltige Verbindungen des Zirkons, berechnet als ZrO₂,
- 5 bis 30 Gew.-% sauerstoffhaltige Verbindungen des Kupfers, berechnet als CuO,
- 0,1 bis 10 Gew.-% sauerstoffhaltige Verbindungen des Molybdäns, berechnet als MoO₃ und,
- 0 bis 10 Gew.-% weiterer Komponenten,
wobei sich die Angaben in Gew.-% auf den trockenen, nicht reduzierten Katalysator beziehen.

2. Verfahren nach Anspruch 1, worin R¹ und R² gleich Wasserstoff sind.

3. Verfahren nach Anspruch 1 oder 2, worin ein Katalysatoren eingesetzt wird, umfassend
- 45 bis 55 Gew.-% sauerstoffhaltige Verbindungen des Nickels, berechnet als NiO,
- 25 bis 35 Gew.-% sauerstoffhaltige Verbindungen des Zirkons, berechnet als ZrO₂,
- 13 bis 20 Gew.-% sauerstoffhaltige Verbindungen des Kupfers, berechnet als CuO,
- 1 bis 3 Gew.-% sauerstoffhaltige Verbindungen des Molybdäns, berechnet als MoO₃ und
- 0 bis 5 Gew.-% weiterer Komponenten,
wobei sich die Angaben in Gew.-% zu 100 Gew.-% ergänzen und sich auf den trockenen, nicht reduzierten Katalysator beziehen.

4. Verfahren nach einem der Ansprüche 1 bis 3, worin der Katalysator in Form eines Festbettkatalysators eingesetzt wird.

5. Verfahren nach einem der Ansprüche 1 bis 4, worin die Hydrierung bei einem Wasserstoffdruck im Bereich von 4 bis 80 bar absolut durchgeführt wird.

6. Verfahren nach einem der Ansprüche 1 bis 5, worin die Hydrierung bei einer Temperatur im Bereich von 100 bis 180°C durchgeführt wird.

7. Verfahren nach einem der Ansprüche 1 bis 6, worin die katalytische Hydrierung kontinuierlich durchgeführt wird.

8. Verfahren nach einem der Ansprüche 1 bis 7, worin in einem weiteren Verfahrensschritt ein bei der katalytischen Hydrierung erhaltenes Reaktionsprodukt zur weiteren Aufreinigung des substituierten oder unsubstituierten Phenylcyclohexans der Formel I destillativ aufgearbeitet wird.

9. Verfahren nach Anspruch 8, worin die destillative Aufarbeitung des Reaktionsproduktes aus der katalytischen Hydrierung im Vakuum bei Absolutdrücken zwischen 1 und 100 mbar durchgeführt wird.

10. Verfahren nach Anspruch 8 oder 9, worin Fraktionen, die bei der destillativen Aufarbeitung des Reaktionsproduktes aus der katalytischen Hydrierung anfallen und die noch Biphenyl der Formel II enthalten, ganz oder teilweise in die Hydrierung zurückgeführt werden.

11. Verfahren nach einem der Ansprüche 1 bis 10, worin die Hydrierung so durchgeführt wird, dass zwischen 90 und 95 % des substituierten oder unsubstituierten Biphenyls der Formel II umgesetzt werden.

## Claims

1. A process for preparing a substituted or unsubstituted phenylcyclohexane of the formula I 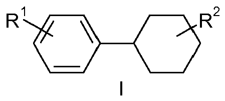 by catalytic hydrogenation of a substituted or unsubstituted biphenyl of the formula II 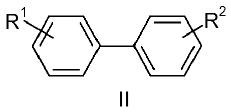 where R¹ is hydrogen and R² is hydrogen or phenyl, or R¹ is a C₁-C₄-alkyl radical and R² is hydrogen, or R¹ and R² are identical and are each phenyl or the same C₁-C₄-alkyl radical, where R¹ and R² are both simultaneously located in the respective ortho, meta or para positions of the two phenyl rings of the biphenyl of the formula II,
in the presence of hydrogen and a catalyst comprising
- from 30 to 70% by weight of oxygen-comprising compounds of nickel, calculated as NiO,
- from 15 to 45% by weight of oxygen-comprising compounds of zirconium, calculated as ZrO₂,
- from 5 to 30% by weight of oxygen-comprising compounds of copper, calculated as CuO,
- from 0.1 to 10% by weight of oxygen-comprising compounds of molybdenum, calculated as MoO₃, and,
- 0 to 10% by weight of further components,
where the figures in % by weight are based on the dry, unreduced catalyst.

2. The process according to claim 1, wherein R¹ and R² are each hydrogen.

3. The process according to claim 1 or 2, wherein a catalyst comprising
- from 45 to 55% by weight of oxygen-comprising compounds of nickel, calculated as NiO,
- from 25 to 35% by weight of oxygen-comprising compounds of zirconium, calculated as ZrO₂,
- from 13 to 20% by weight of oxygen-comprising compounds of copper, calculated as CuO,
- from 1 to 3% by weight of oxygen-comprising compounds of molybdenum, calculated as MoO₃, and
- from 0 to 5% by weight of further components,
where the figures in % by weight add up to 100% by weight and are based on the dry, unreduced catalyst, is used.

4. The process according to any of claims 1 to 3, wherein the catalyst is used in the form of a fixed-bed catalyst.

5. The process according to any of claims 1 to 4, wherein the hydrogenation is carried out at a hydrogen pressure in the range from 4 to 80 bar absolute.

6. The process according to any of claims 1 to 5, wherein the hydrogenation is carried out at a temperature in the range from 100 to 180°C.

7. The process according to any of claims 1 to 6, wherein the catalytic hydrogenation is carried out continuously.

8. The process according to any of claims 1 to 7, wherein, in a further process step, a reaction product obtained in the catalytic hydrogenation is worked up by distillation to purify the substituted or unsubstituted phenylcyclohexane of the formula I further.

9. The process according to claim 8, wherein the work-up by distillation of the reaction product from the catalytic hydrogenation is carried out under reduced pressure at absolute pressures in the range from 1 to 100 mbar.

10. The process according to claim 8 or 9, wherein fractions which are obtained in the work-up by distillation of the reaction product from the catalytic hydrogenation and still comprise biphenyl of the formula II are recirculated in their entirety or in part to the hydrogenation.

11. The process according to any of claims 1 to 10, wherein the hydrogenation is carried out so that from 90 to 95% of the substituted or unsubstituted biphenyl of the formula II is reacted.

## Revendications

1. Procédé de fabrication d'un phénylcyclohexane substitué ou non substitué de formule I 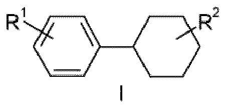 par hydrogénation catalytique d'un biphényle substitué ou non substitué de formule II 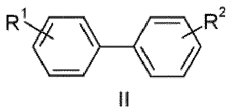 dans lesquelles R¹ représente hydrogène et R² représente hydrogène ou phényle, ou R¹ représente un radical alkyle en C₁-C₄ et R² représente hydrogène, ou R¹ et R² sont identiques et représentent chacun phényle ou le même radical alkyle en C₁-C₄, R¹ et R² se trouvant tous les deux simultanément aux positions ortho, méta ou para respectives des deux cycles phényle du biphényle de formule II,
en présence d'hydrogène et d'un catalyseur, comprenant
- 30 à 70 % en poids de composés oxygénés de nickel, calculés en tant que NiO,
- 15 à 45 % en poids de composés oxygénés de zirconium, calculés en tant que ZrO₂,
- 5 à 30 % en poids de composés oxygénés de cuivre, calculés en tant que CuO,
- 0,1 à 10 % en poids de composés oxygénés de molybdène, calculés en tant que MoO₃, et
- 0 à 10 % en poids de composants supplémentaires, les données en % en poids se rapportant au catalyseur sec non réduit.

2. Procédé selon la revendication 1, dans lequel R¹ et R² représentent l'hydrogène.

3. Procédé selon la revendication 1 ou 2, dans lequel un catalyseur comprenant
- 45 à 55 % en poids de composés oxygénés de nickel, calculés en tant que NiO,
- 25 à 35 % en poids de composés oxygénés de zirconium, calculés en tant que ZrO₂,
- 13 à 20 % en poids de composés oxygénés de cuivre, calculés en tant que CuO,
- 1 à 3 % en poids de composés oxygénés de molybdène, calculés en tant que MoO₃, et
- 0 à 5 % en poids de composants supplémentaires, les données en % en poids s'additionnant pour atteindre 100 % en poids et se rapportant au catalyseur sec non réduit,
est utilisé.

4. Procédé selon l'une quelconque des revendications 1 à 3, dans lequel le catalyseur est utilisé sous la forme d'un catalyseur à lit fixe.

5. Procédé selon l'une quelconque des revendications 1 à 4, dans lequel l'hydrogénation est réalisée à une pression d'hydrogène dans la plage allant de 4 à 80 bar absolu.

6. Procédé selon l'une quelconque des revendications 1 à 5, dans lequel l'hydrogénation est réalisée à une température dans la plage allant de 100 à 180 °C.

7. Procédé selon l'une quelconque des revendications 1 à 6, dans lequel l'hydrogénation catalytique est réalisée en continu.

8. Procédé selon l'une quelconque des revendications 1 à 7, dans lequel, lors d'une étape de procédé supplémentaire, un produit de réaction obtenu lors de l'hydrogénation catalytique est traité par distillation pour la purification supplémentaire du phénylcyclohexane substitué ou non substitué de formule I.

9. Procédé selon la revendication 8, dans lequel le traitement par distillation du produit de réaction issu de l'hydrogénation catalytique est réalisé sous vide à des pressions absolues comprises entre 1 et 100 mbar.

10. Procédé selon la revendication 8 ou 9, dans lequel des fractions formées lors du traitement par distillation du produit de réaction issu de l'hydrogénation catalytique et contenant encore du biphényle de formule II sont recyclées en totalité ou en partie dans l'hydrogénation.

11. Procédé selon l'une quelconque des revendications 1 à 10, dans lequel l'hydrogénation est réalisée de sorte qu'entre 90 et 95 % du biphényle substitué ou non substitué de formule II soit transformé.
